# EUROPEAN PATENT SPECIFICATION

(11) **EP 3 313 476 B1**
(45) Date of publication and mention of the grant of the patent: **21.08.2019**
(21) Application number: 16729940.3
(22) Date of filing: 20.06.2016
(51) Int. Cl.: A61M 5/178, A61M 5/315

(54) **BACKSTOP FOR A SYRINGE**
ANSCHLAG FÜR EINE SPRITZE
BUTÉE ARRIÈRE POUR UNE SERINGUE

(30) Priority: 25.06.2015 US 201562184545 P
(43) Date of publication of application: 02.05.2018
(73) Proprietor: Merck Sharp & Dohme B.V., 2031 BN Haarlem (NL)
(72) Inventor: BROEKE, Dennis, 2031 BN Haarlem (NL); DE JONG, Sander H.W., 2031 BN Haarlem (NL); DE WAAL MALEFIJT, Jacobus A., 2031 BN Haarlem (NL)
(74) Representative: Böhles, Elena
(86) International application number: PCT/EP2016/064169
(87) International publication number: WO 2016/207108

(56) References cited:
- US-A1- 2004 254 539
- US-A1- 2007 088 284
- US-A1- 2011 190 628
- US-A1- 2013 144 220
- US-B1- 6 296 625

## Description

### BACKGROUND OF THE INVENTION

It is well known to store certain medicine or pharmaceutical products in lyophilized or powdered form to improve the usable life of the products. Such medicine may lose stability, effectiveness and/or potency once mixed into a solution, over time.

Reconstitution is the known process of mixing medicine in dried, powdered, or lyophilized form with a liquid (often called the diluent) prior to administering or providing the medicine to a patient. Reconstitution is often accomplished using a vial containing medicine *(e.g.,* a vaccine) and a syringe containing the diluent. At least some or the entire contents of the syringe is injected into the vial. The vial is then gently agitated to dissolve the medicine within the vial. The dissolved medicine is then withdrawn into the same syringe used to inject the diluent into the vial. The dissolved medicine in the syringe is then administered to or injected into a patient.

Although reconstitution is a widely used and valuable process, certain aspects of the process could be improved. For example, healthcare professionals and/or patients often complain that the step of withdrawing the dissolved medicine from the vial and into the syringe can be more difficult than desired. To withdraw the dissolved medicine into the syringe, a plunger must be partially withdrawn from, or at least partially pulled out of, the syringe, to pull the dissolved product/diluent into the barrel of the syringe.

One undesirable, yet often encountered, outcome is that the entire plunger is inadvertently pulled out of or separated from a barrel of the syringe when the dissolved medicine is withdrawn from the vial and into the syringe. This outcome has numerous disadvantages, including loss of sterility of the product, and a longer wait time for the patient, because the medicine must be reconstituted again.

US 2013/0144220 A1, US 6,296,625 B1, and US 2011/0190628 A1, relating to backstops for syringes, represent the closest prior art.

The present invention overcomes the above-identified disadvantages of the prior art, and accomplishes the above and other objectives.

### BRIEF SUMMARY

The invention provides a backstop for a syringe according to claim 1. Further aspects and preferred embodiments are defined in the dependent claims. Aspects, embodiments and examples of the present disclosure that do not fall under the scope of the appended claims do not form part of the invention and are merely provided for illustrative purposes. Furthermore, the methods presented in the present description are provided for illustrative purposes only and do not form part of the present invention.

In one embodiment, the present disclosure is directed to a backstop for a syringe. The backstop can include a base having a first surface and an opposing second surface. A skirt can extend outwardly from the second surface of the base. An opening can extend through a geometric center of the base. The opening can be surrounded by the skirt. A first extension can extend generally perpendicularly from the second surface at an outer periphery of the base. A second extension can extend generally perpendicularly from the second surface at the outer periphery of the base. The second extension can be diametrically opposed to the first extension.

In another embodiment, the present disclosure is directed to a combination including a syringe and a backstop. The syringe may include a barrel and a flange. The flange may extend outwardly from the barrel. The backstop may be configured to be attached to at least a portion of the flange of the syringe. The backstop can include a base having a first surface and an opposing second surface. A skirt can extend outwardly from the second surface of the base. An opening can extend through a geometric center of the base. The opening can be surrounded by the skirt. A first extension can extend generally perpendicularly from the second surface at an outer periphery of the base. A second extension can extend generally perpendicularly from the second surface at the outer periphery of the base. The second extension can be position diametrically opposed to the first extension.

### BRIEF DESCRIPTION OF THE SEVERAL VIEWS OF THE DRAWINGS

The foregoing summary, as well as the following detailed description of the invention, will be better understood when read in conjunction with the appended drawings. For the purpose of illustrating the invention, there are shown in the drawings various illustrative embodiments. It should be understood, however, that the invention is not limited to the precise arrangements and instrumentalities shown. In the drawings:
Fig. 1 is a perspective view of a combination according to an embodiment of the present disclosure, wherein the combination is shown in a fully assembled configuration;
Fig. 2 is a partially exploded perspective view of the combination shown in Fig. 1;
Fig. 3 is a fully exploded perspective view of the combination shown in Fig. 1;
Fig. 4 is a bottom perspective view of a backstop according to an embodiment of the present disclosure;
Fig. 5 is a top perspective view of the backstop shown in Fig. 4;
Fig. 6 is a top plan view of the backstop shown in Fig. 4;
Fig. 6A is a cross-sectional side elevation view of the backstop shown in Fig. 4, taken along line B-B of Fig. 6;
Fig. 7 is a side elevation view of the backstop shown in Fig. 4;
Fig. 8 is an enlarged side elevation view of the backstop shown in Fig. 4, wherein at least a portion of the backstop is shown as deflecting;
Fig. 9 is another enlarged side elevation view of the backstop shown in Fig. 4; wherein at least a portion of the backstop is shown as deflecting; and
Fig. 10 is a side elevation view of an assembly line for attaching the backstop to at least a portion of a syringe.

### DETAILED DESCRIPTION

Certain terminology is used in the following description for convenience only and is not limiting. The words "bottom" and "top" designate directions in the drawings to which reference is made. Unless specifically set forth herein, the terms "a," "an" and "the" are not limited to one element but instead should be read as meaning "at least one." The terminology includes the words noted above, derivatives thereof and words of similar import.

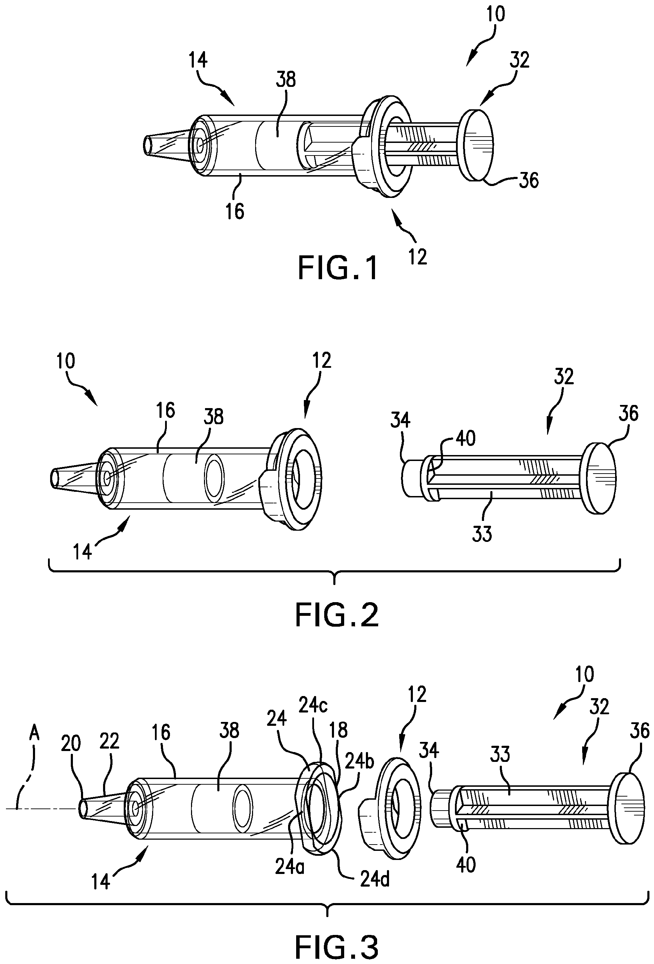

Referring to the drawings in detail, wherein like numerals indicate like elements throughout, Figs. 1-10 illustrate a combination 10, or individual elements thereof, including a backstop 12 and a syringe 14, a method of using the backstop 12, and a method of attaching or applying the backstop 12 to at least a portion of the syringe 14. The term "backstop" is defined herein to broadly encompass any apparatus capable of being at least temporarily attached to at least a portion of the syringe 14 and accomplishing the functionality described herein. This term "backstop" is not intended to limit the scope of the claims or require particular structure or particular functionality. Instead, the term "backstop" is used for convenience only and can be interchanged with other generic terms.

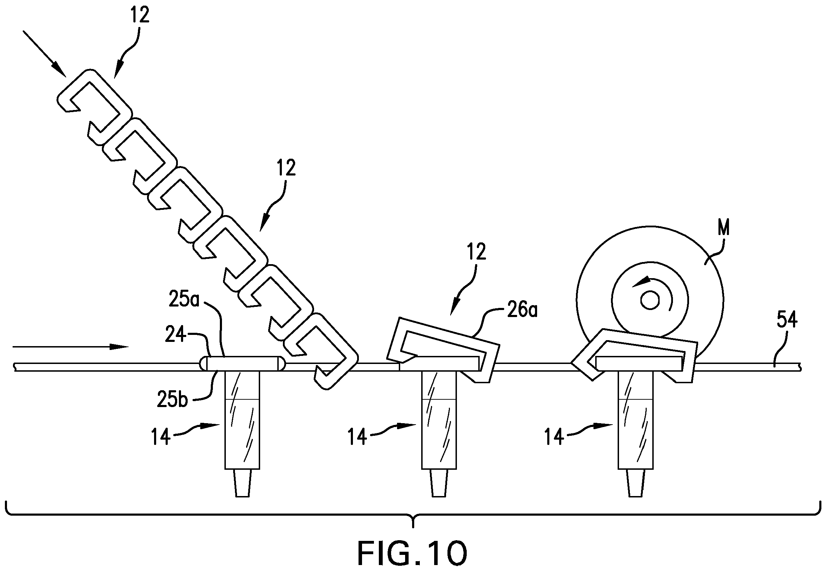

The syringe 14 can include a generally cylindrical barrel 16 extending between a proximal end 18 and a distal end 20 of the syringe 14 (see Fig. 3). A tip or nozzle 22 can be located at the distal end, and a flange 24 can be located at the proximal end. The flange 24 can extend outwardly and/or at least generally or exactly perpendicularly to a longitudinal axis A of the barrel 16 (see Fig. 3). The flange 24 of the syringe 14 can include at least a generally or entirely flat or planar top and bottom surfaces 25a, 25b (see Fig. 10). The syringe 14 may be formed of glass or a polymeric material, for example.

As shown in Fig. 3, the flange 24 can include diametrically opposed flat, planar or linear sides 24a, 24b and diametrically opposed arcuate sides 24c, 24d. One of the flat sides 24a, 24b can be spaced between the two arcuate sides 24c, 24d around a periphery of the flange 24. In such an embodiment, at least a portion or point of each flat side 24a, 24b may be coplanar with at least a portion of an exterior surface of the barrel 16 of the syringe 14. Alternatively, the flange 24 can be entirely circular or ovular when viewed from above and/or below.

A plunger 32 can be sized, shaped and/or configured to be inserted into and/or received by at least a portion of the barrel 16 of the syringe 14. The plunger 32 can include a distal end 34. an opposing proximal end 36, and a shaft 33 extending therebetween. The distal end 34 can be configured to engage and/or receive at least a portion of a stopper 38. The stopper 38 can be formed of rubber. The proximal end 36 can extend radially outwardly beyond the shaft 33 and/or the distal end 34 to provide a handle to a user. In one embodiment, one or more projections 40 can extend outwardly and/or at least generally or exactly perpendicularly from at least a portion (*e*.*g*., the shaft 33) of the plunger 32, such that the shaft 33 has an x-shaped cross section. As described in detail below, the backstop 12 may be sized, shaped and/or configured to preclude unintentional withdrawal of the plunger 32 and/or the stopper 38 from the syringe 14.

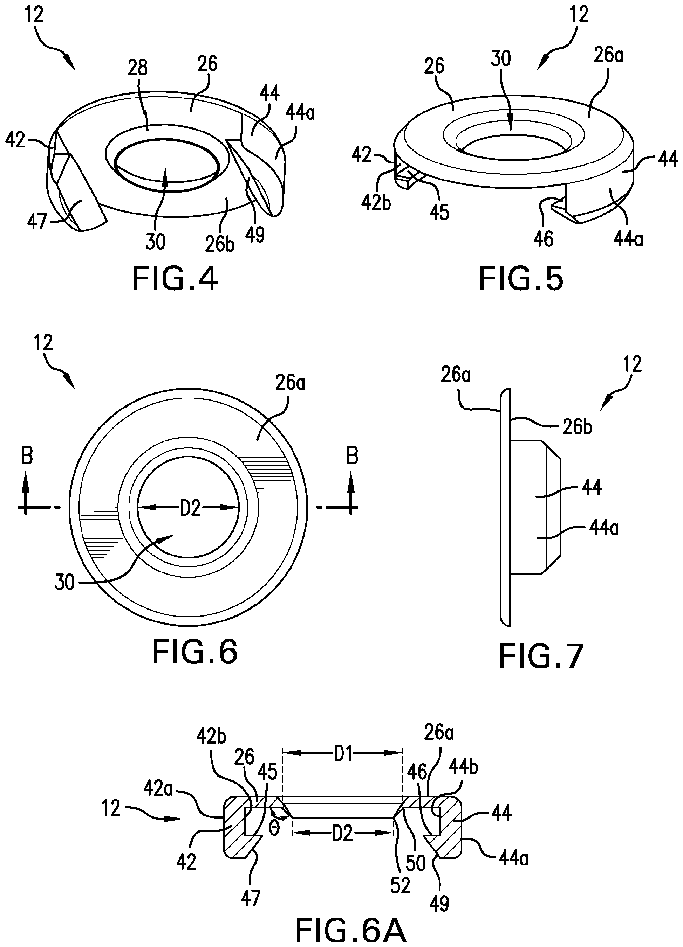

Referring to Figs. 4-7, the backstop 12 can include a base 26 including a first or top surface 26a and an opposing second or bottom surface 26b. Each of the first and second surfaces 26a, 26b can be flat or planar and extend generally, if not exactly, parallel to each other. However, the first and second surfaces 26a, 26b of the base 26 are not so limited. For example, the first and second surfaces 26a, 26b of the base 26 may be flexible or resilient, such that both surfaces 26a, 26b can be bent, at least slightly, and return to the original, flat configuration, as described in more detail below.

A skirt 28 can extend at least slightly outwardly and/or downwardly from the second surface 26b of the base 26. As shown in Fig. 6A, in a non-deflected or relaxed configuration, an outer or exterior surface of the skirt 28 can form an angle θ with respect to the second surface 26b of the base 26. The angle θ can be in the range of approximately 95-175 degrees. More particularly, the angle θ can be approximately 130-140 degrees, or exactly 135 degrees.

The skirt 28 can include a first end 50 at the second surface 26b of the base 26 and a second end 52 spaced apart from the first end 50. The skirt 28 can be formed of the same or a different material than the remainder of the backstop 12. For example, the skirt 28 may be formed of an elastic, resilient material, while the remainder of the backstop 12 may be formed of a light-weight, high-strength material, such as a polymeric material.

An opening 30 can extend through the base 26 of the backstop. More particularly, the opening 30 can extend from and through the first surface 26a of the base 26 to and through the second surface 26b of the base 26. In one embodiment, the opening 30 can be generally or exactly circular when viewed from above or below. A first diameter D1 of the opening 30 at the first end 50 of the skirt 28 is greater than a second diameter D2 of the opening 30 at the second end 52 of the skirt 28. In one embodiment, the first diameter D1 of the opening 30 is at least slightly greater than an outer diameter of the shaft 33 and/or the distal end 34 of the plunger 32, while the second diameter D2 of the opening 30 is slightly less than the outer diameter of the shaft 33 and/or the distal end 34 of the plunger 32. With such a configuration, the skirt 28 will be elastically deformed when the distal end 34 of the plunger 32 is inserted into and passed through the skirt 28, then deformed into a squared opening around the x-shaped cross section of the plunger 32, such that inner edges of the skirt 28 block the distal end 34 of the plunger 32 from being withdrawn past the skirt 28.

The opening 30 can be surrounded completely by or at least partially defined by the skirt 28. Thus, in one embodiment, the skirt 28 can form a relatively short or truncated cone, such that the skirt 28 extends completely around *(e.g.,* 360 degrees) the opening 30. However, the skirt 28 is not limited to extending completely around the opening 30. For example, the skirt 28 may be formed of two or more (*e*.*g*., four) separate and spaced-apart components. In one embodiment, the skirt 28 may include a plurality of spaced-apart slits or fingers that extend from the second end 52 of the skirt 28 toward (but not necessarily reaching) the first end 50 of the skirt 28. The slits may provide increased flexibility and/or functionality to the skirt 28. For example, inward edges of the slits may engage or catch at least a portion of the projection(s) 40, the shaft 33 and/or the distal end 34 of the plunger 32 as the plunger is being pulled away from the syringe 14.

A first extension 42 can extend downwardly and/or at least generally or exactly perpendicularly from the second surface 26b at an outer periphery or circumference of the base 26. A second extension 44 can extend downwardly and/or at least generally or exactly perpendicularly from the second surface 26b at the outer periphery or circumference of the base 26. The second extension 44 can be spaced-apart a predetermined distance from the first extension 42. In one embodiment, the second extension 44 is at least generally or exactly diametrically opposed to the first extension 42. As shown in Fig. 6A, the first and second extensions 42, 44 of the backstop 12 extend further from the second surface 26b of the base 26 than the skirt 28 and are positioned radially outwardly with respect to the skirt 28.

The first extension 42 can include a first or exterior surface 42a and an opposing second or interior surface 42b. The first surface 42a of the first extension 42 can be at least generally or entirely curved (*e*.*g.*, concave) or arcuate. The second surface 42b of the first extension 42 can be at least generally or entirely flat or planar. A first ledge 45 can extend at least generally or exactly perpendicularly to the second surface 42b of the first extension 42 and toward the opening 30. The first ledge 45 can be spaced-apart from both the second surface 26b of the base 26 and an opposing free end of the first extension 42. A first slanted or angled surface or face 47 may connect or extend from a free edge of the first ledge 45 to a free end of the first extension 42.

The second extension 44 can include a first or exterior surface 44a and an opposing second or interior surface 44b. The first surface 44a of the second extension 44 can be at least generally or entirely curved (*e*.*g*., concave) or arcuate. The second surface 44b of the second extension 44 can be at least generally or entirely flat or planar. A second ledge 46 can extend at least generally or exactly perpendicularly to the second surface 44b of the second extension 44. The second ledge 46 can be spaced-apart from both the second surface 26b of the base 26 and an opposing free end of the second extension 44 and toward the opening 30. A second slanted or angled surface or face 49 may connect or extend from a free edge of the second ledge 46 to a free end of the second extension 44.

In one embodiment, when the backstop 12 is properly attached to the syringe 14, at least a portion of the second surface 42b, 44b of each of the first and second extensions 42, 44 contacts, engages and/or faces at least a portion of the flange 24 of the syringe 14. The complimentary size, shape and configuration of the backstop 12 and the flange 24 of the syringe 14 can prevent or at least inhibit the backstop 12 from inadvertently rotating with respect to the syringe 14 when the backstop 12 is properly attached to the flange 24 of the syringe 14. Such an arrangement is also beneficial because there is no need to change pre-existing packaging and/or the configuration of existing syringe flanges to accommodate or work with the backstop 12. The ledges 45, 46 may engage the flange 24 of the syringe 14 to create latching engagement between the backstop 12 and the syringe 14, thereby preventing the backstop 12 from being accidentally disengaged from the syringe 14.

The opening 30 and/or the skirt 28 can be sized, shaped and/or configured to permit at least a portion of the plunger 32 to pass therethrough. The opening 30 and/or the skirt 28 can also be sized, shaped and/or configured to prevent, or at least inhibit, at least a portion of the plunger 32 from passing therethrough. For example, the opening 30 and/or the skirt 28 can permit at least the distal end 34 of the plunger 32 to be inserted into a cavity of the barrel 16 of the syringe 14. During this process, at least a portion of the plunger 32 may deflect as least a portion of the skirt 28 radially outward or away from the longitudinal axis A of the syringe 14. However, when a user attempts to remove the plunger 32 from the cavity of the barrel 16 of the syringe 14, at least a portion of the skirt 28, such as the second end 52 thereof, can engage at least a portion of the plunger 32, such as the projection(s) 40 thereof. As a result, the plunger 32 is prevented from being removed from the barrel 16 of the syringe 14, or at least the process is made to be more difficult by the existence of the skirt 28.

In one embodiment, rotation of the plunger 32 within the opening 30 of the backstop 12 can be precluded or at least reduced based on engagement of at least a portion of the skirt 28 with at least a portion of the plunger 32. In an alternative embodiment, such as when the skirt 28 is formed via a continuous opening, the plunger 32 can be permitted to rotate 360 degrees when at least a portion of the plunger 32 is inserted into the opening 30 of the backstop 12.

In one embodiment, the backstop 12 can be designed to be at least partially flexible or bendable. Such a design can allow the backstop 12 to more easily be attached to at least a portion of the flange 24 of the syringe 14, and/or more effectively permit or inhibit movement of the plunger 32 into and out of the syringe 14 when the backstop 12 is attached to the syringe 14.

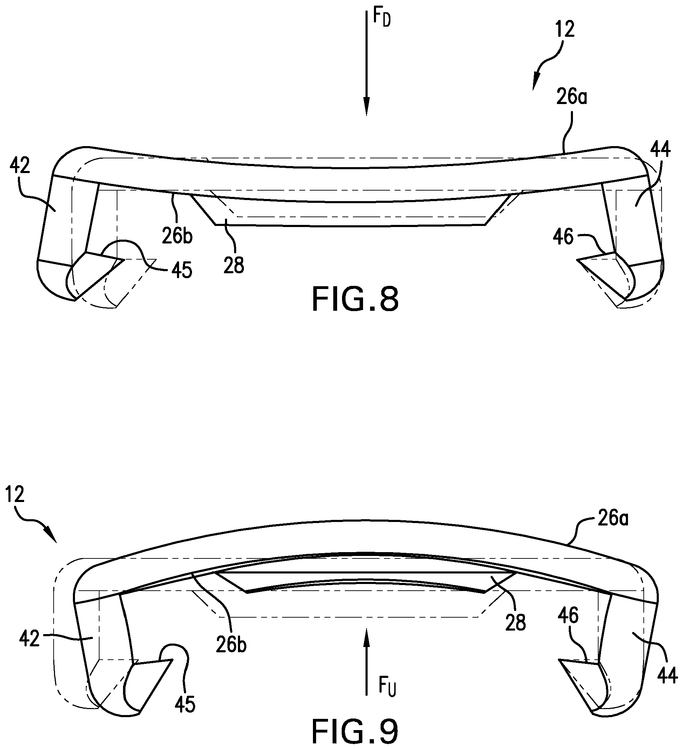

As shown in Fig. 8, the first surface 26a of the base 26 can be reconfigurable to at least a slightly concave position and the second surface 26b of the base 26 may move to at least a slightly convex position. These positions can be the result of a downward force F_{D} being applied to at least a portion of the first surface 26a of the base 26. This downward force F_{D} can be created by a user pressing downward (*e*.*g*., with a finger or thumb) onto the base 26 of the backstop 12. As a result of this downward force F_{D} and the corresponding reconfiguration of the first and second surfaces 26a, 26b of the base 26, the first and second extensions 42, 44 can move (*e*.*g*., pivot) at least slightly outwardly away from the skirt 28. Such movement of the first and second extensions 42, 44 can allow at least the first and second ledges 45, 46 to pass over and then engage at least a portion of the flange 24 of the syringe 14. As a result, in one embodiment, the reconfigurable nature of the base 26 can allow the base 26 to be attached more easily to the flange 24 of the syringe 14.

As shown in Fig. 9, the first surface 26a of the base 26 can be reconfigurable to at least a slightly convex position and the second surface 26b of the base 26 may move to at least a slightly concave position. These positions can be the result of an upward force F_{U} being applied to at least a portion of the second surface 26b of the base 26 and/or the skirt 28. This upward force F_{U} can be created by a user attempting to pull the plunger 32 out of or away from the syringe 14. As a result of this upward force F_{U} and the corresponding reconfiguration of the first and second surfaces 26a, 26b of the base 26, at least the second end 52 and/or a free end of the slits of the skirt 28 can move at least slightly inwardly toward a geometric center of the opening 30. Accordingly, in one embodiment, the interior or second diameter D2 of the opening 30 is a least slightly reduced, thereby increasing the latching effect of the skirt 28 on at least a portion of the plunger 32 and making it at least more difficult to remove the plunger 32 from the syringe 14. In addition or alternatively, as a result of this upward force F_{U} and the corresponding reconfiguration of the first and second surfaces 26a, 26b of the base 26, the first and second extensions 42, 44 can move (*e*.*g*., pivot) at least slightly inwardly toward the skirt 28. Such movement of the first and second extensions 42, 44 can allow at least the first and second ledges 45, 46 to more securely engage or "hook" at least a portion (*e*.*g*., an underside) of the flange 24 of the syringe 14, thereby preventing and/or increasing the difficulty of separating the backstop 12 and the plunger 32 from the syringe 14.

The design of the backstop 12 provides for efficient manufacturing and/or efficient application of the backstop 12 to the syringe 14. As shown in Fig. 10, at least one or a plurality or series of interconnected backstops 12 can be placed near the flange 24 of a syringe 14. The syringe(s) 14 may be moved on a conveyor belt or other apparatus that moves the syringe 14 or a plurality of spaced-apart syringes, e.g., from left to right as shown in Fig. 10. Each syringe 14 may be suspended from a conveyor belt 54. A leading edge of the flange 24 of each syringe 14 may contact or engage the second extension 44, for example, of the backstop 12. The backstop(s) 12 may be fed or flow from a sorting hopper (not shown). As the syringe 14 continues to move (*e*.*g*., to the right), the second ledge 46 of the second extension 44 contacts an underside of the flange 24 of the syringe 14. As the syringe 14 continues to move (*e.g*., to the right), a roller or press M (*e*.*g*., rotating counterclockwise) may apply a downward force F_{D} to the first surface 26a of the base 26 of the backstop 12, until the first ledge 45 of the first extension 42 of the backstop 12 is pushed downwardly and beyond an opposing side of the flange 24 of the syringe 14. Once the backstop 12 is properly attached to the syringe 14, the plunger 32 may be inserted into the syringe 14, as shown in Figs. 1 and 2.

It will be appreciated by those skilled in the art that changes could be made to the embodiments described above without departing from the broad inventive concept thereof. It is understood, therefore, that this invention is not limited to the particular embodiments disclosed, but it is intended to cover modifications within the scope of the present invention as defined by the appended claims.

## Claims

1. A backstop for a syringe (12), the backstop comprising:
a base (26) including a first surface (26a) and an opposing second surface (26b);
a skirt (28) extending outwardly from the second surface of the base;
an opening (30) extending through the base, the opening being at least generally surrounded by the skirt;
a first extension (42) extending generally perpendicularly from the second surface of the base at an outer periphery of the base, the first extension including a first surface (42a) and an opposing second surface (42b), a first ledge (45) extending outwardly from the second surface of the first extension; and
a second extension (44) extending generally perpendicularly from the second surface of the base at the outer periphery of the base, the second extension being diametrically opposed to the first extension, the second extension including a first surface (44a) and an opposing second surface (44b), a second ledge (46) extending outwardly from the second surface of the second extension;
wherein the skirt includes a first end (50) at the second surface of the base and a second end (52) spaced apart therefrom;
**characterised in that** a first diameter of the opening (D1) at the first end of the skirt is greater than a second diameter (D2) of the opening at the second end of the skirt; optionally wherein the skirt is formed of an elastic material.

2. The backstop of claim 1, wherein the first surface of the first extension is at least generally arcuate and the second surface of the first extension is at least generally planar.

3. The backstop of claim 1 or 2, wherein the first ledge extends generally perpendicularly to the second surface of the first extension, and/or wherein the first surface of the second extension is at least generally arcuate and the second surface of the second extension is at least generally planar.

4. The backstop of claim any of claims 1-3, wherein the second ledge extends generally perpendicularly to the second surface of the second extension, and/or wherein at least a portion of the second surface of each of the first and second extensions contacts at least a portion of a planar side of a flange (24) of the syringe.

5. The backstop of any of claims 1-4, wherein the first and second extensions extend further from the second surface than the skirt.

6. A combination comprising:
a syringe (14) including a barrel (16) and a flange (24), the flange extending outwardly from the barrel; and
the backstop according to claim 1 configured to be attached to at least a portion of the flange of the syringe, wherein the first ledge of the first extension extends outwardly from the second surface of the first extension, at least a portion of the first ledge contacting at least a portion of the flange of the syringe, and wherein the second ledge of the second extension extends outwardly from the second surface of the second extension, at least a portion of the second ledge contacting at least a portion of the flange of the syringe; wherein optionally the combination further comprsing a plunger (32) configured to be inserted into at least a portion of the barrel of the syringe, the backstop preventing the plunger from being separated from the syringe.

7. The combination of claim 6, wherein the plunger includes a distal end (20) and at least one projection (40) extending outwardly therefrom, the distal end being configured to engage at least a portion of a stopper, at least a portion of the backstop contacting at least a portion of the projection of the plunger.

8. The combination of claims 6 or 7, wherein the first surface of the first extension is at least generally arcuate and the second surface of the first extension is at least generally planar.

9. The combination of any of claims 6 to 8, wherein the first ledge extends generally perpendicularly to the second surface of the first extension.

10. The combination of any of claims 6 to 9, wherein the first surface of the second extension is at least generally arcuate and the second surface of the second extension is at least generally planar.

11. The combination of any of claims 6 to 10, wherein the second ledge extends generally perpendicularly to the second surface of the second extension.

12. The combination of any of claims 6 to 11, wherein the flange of the syringe includes a first planar side (24a), a diametrically opposed second planar side (24b), a first arcuate side (24c) and an opposing second arcuate side (24d), and optionally wherein at least a portion of the second surface of each of the first and second extensions contacts at least a portion of one of the first and second planar sides of a flange of the syringe.

## Patentansprüche

1. Anschlag für eine Spritze (12), wobei der Anschlag umfasst:
eine Basis (26) mit einer ersten Oberfläche (26a) und einer gegenüberliegenden zweiten Oberfläche (26b),
eine Schürze (28), die sich von der zweiten Oberfläche der Basis nach außen erstreckt,
einer Öffnung (30), die durch die Basis reicht, wobei die Öffnung wenigstens im Allgemeinen von der Schürze eingefasst ist,
einer ersten Verlängerung (42) die sich im Allgemeinen rechtwinklig von der zweiten Oberfläche der Basis an einem äußeren Rand der Basis erstreckt, wobei die erste Verlängerung umfasst: eine erste Oberfläche (42a) und eine gegenüberliegende zweite Oberfläche (42b), einen ersten Vorsprung (45), der sich von der zweiten Oberfläche der ersten Verlängerung nach außen erstreckt, und
eine zweite Verlängerung (44), die sich im Allgemeinen rechtwinklig von der zweiten Oberfläche der Basis am äußeren Rand der Basis erstreckt, wobei die zweite Verlängerung diametral der ersten Verlängerung entgegengesetzt ist, die zweite Verlängerung umfasst: eine erste Oberfläche (44a) und eine gegenüberliegende zweite Oberfläche (44b), einen zweiten Vorsprung (46), der sich von der zweiten Oberfläche der zweiten Verlängerung nach außen erstreckt,
wobei die Schürze ein erstes Ende (50) an der zweiten Oberfläche der Basis und, im Abstand dazu, ein zweites Ende (52) umfasst,
**dadurch gekennzeichnet, dass** ein erster Durchmesser der Öffnung (D1) am ersten Ende der Schürze größer ist als ein zweiter Durchmesser (D2) der Öffnung am zweiten Ende der Schürze, gegebenenfalls wobei die Schürze aus einem elastischen Material gebildet ist.

2. Anschlag nach Anspruch 1, wobei die erste Oberfläche der ersten Verlängerung wenigstens im Allgemeinen bogenförmig ist und die zweite Oberfläche der ersten Verlängerung wenigstens im Allgemeinen planar ist.

3. Anschlag nach Anspruch 1 oder 2, wobei der erste Vorsprung sich im Allgemeinen rechtwinklig zur zweiten Oberfläche der ersten Verlängerung erstreckt, und/oder wobei die erste Oberfläche der zweiten Verlängerung wenigstens im Allgemeinen bogenförmig ist und die zweite Oberfläche der zweiten Verlängerung wenigstens im Allgemeinen planar ist.

4. Anschlag nach einem der Ansprüche 1-3, wobei der zweite Vorsprung sich im Allgemeinen rechtwinklig zur zweiten Oberfläche der zweiten Verlängerung erstreckt, und/oder wobei wenigstens ein Teil der zweiten Oberfläche von jeweils der ersten und der zweiten Verlängerung wenigstens einen Teil einer planaren Seite eines Flansches (24) der Spritze berührt.

5. Anschlag nach einem der Ansprüche 1-4, wobei die erste und die zweite Verlängerung sich weiter von der zweiten Oberfläche aus erstrecken als die Schürze.

6. Kombination, umfassend:
eine Spritze (14), die einen Zylinder (16) und einen Flansch (24) umfasst, wobei der Flansch sich vom Zylinder nach außen erstreckt, und
den Anschlag gemäß Anspruch 1, der konfiguriert ist, um an wenigstens einen Teil des Flansches der Spritze befestigt zu werden, wobei der erste Vorsprung der ersten Verlängerung sich von der zweiten Oberfläche der ersten Verlängerung nach außen erstreckt, wenigstens ein Teil des ersten Vorsprungs wenigstens einen Teil des Flansches der Spritze berührt, und wobei der zweite Vorsprung der zweiten Verlängerung sich von der zweiten Oberfläche der zweiten Verlängerung nach außen erstreckt, wenigstens ein Teil des zweiten Vorsprungs wenigstens einen Teil des Flansches der Spritze berührt, wobei gegebenenfalls die Kombination ferner einen Kolben (32) umfasst, der konfiguriert ist, um in wenigstens einen Teil des Zylinders der Spritze eingesetzt zu werden, wobei der Anschlag verhindert, dass der Kolben von der Spritze getrennt wird.

7. Kombination nach Anspruch 6, wobei der Kolben ein distales Ende (20) und wenigstens eine Auskragung (40), die sich davon nach außen erstreckt, umfasst, wobei das distale Ende konfiguriert ist, um in wenigstens einen Teil eines Stoppers zu greifen, wobei wenigstens ein Teil des Anschlags wenigstens einen Teil der Auskragung des Kolbens berührt.

8. Kombination nach den Ansprüchen 6 oder 7, wobei die erste Oberfläche der ersten Verlängerung wenigstens im Allgemeinen bogenförmig ist und die zweite Oberfläche der ersten Verlängerung wenigstens im Allgemeinen planar ist.

9. Kombination nach einem der Ansprüche 6 bis 8, wobei der erste Vorsprung sich im Allgemeinen rechtwinklig zur zweiten Oberfläche der ersten Verlängerung erstreckt.

10. Kombination nach einem der Ansprüche 6 bis 9, wobei die erste Oberfläche der zweiten Verlängerung wenigstens im Allgemeinen bogenförmig ist und die zweite Oberfläche der zweiten Verlängerung wenigstens im Allgemeinen planar ist.

11. Kombination nach einem der Ansprüche 6 bis 10, wobei der zweite Vorsprung sich im Allgemeinen senkrecht zur zweiten Oberfläche der zweiten Verlängerung erstreckt.

12. Kombination nach einem der Ansprüche 6 bis 11, wobei der Flansch der Spritze eine erste planare Seite (24a), eine diametral entgegengesetzte zweite planare Seite (24b), eine erste bogenförmige Seite (24c) und eine gegenüberliegende zweite bogenförmige Seite (24d) umfasst, und gegebenenfalls wobei wenigstens ein Teil der zweiten Oberfläche von jeweils der ersten und der zweiten Verlängerung wenigstens einen Teil von einer der ersten und zweiten planaren Seiten eines Flansches der Spritze berührt.

## Revendications

1. Butée arrière pour une seringue (12), la butée arrière comprenant :
une base (26) incluant une première surface (26a) et une deuxième surface opposée(26b) ;
une jupe (28) s'étendant vers l'extérieur à partir de la deuxième surface de la base ;
une ouverture (30) s'étendant à travers la base, l'ouverture étant au moins généralement entourée par la jupe ;
une première extension (42) s'étendant généralement de manière perpendiculaire à partir de la deuxième surface de la base, au niveau d'une périphérie externe de la base, la première extension incluant une première surface (42a) et une deuxième surface opposée (42b), un premier rebord (45) s'étendant vers l'extérieur de la deuxième surface de de la première extension ; et
une deuxième extension (44) s'étendant généralement de manière perpendiculaire à partir de la deuxième surface de la base, au niveau de la périphérie externe de la base, la deuxième extension étant diamétralement opposée à la première extension, la deuxième extension incluant une première surface (44a) et une deuxième surface opposée (44b), un deuxième rebord (46) s'étendant vers l'extérieur de la deuxième surface de la deuxième extension ;
dans laquelle la jupe inclut une première extrémité (50) au niveau de la deuxième surface de la base, et une deuxième extrémité (52) espacée de celle-ci ;
**caractérisée en ce qu'**un premier diamètre de l'ouverture (D1) au niveau de la première extrémité de la jupe est supérieur à un deuxième diamètre (D2) de l'ouverture au niveau de la deuxième extrémité de la jupe ; dans laquelle la jupe est optionnellement formée à partir d'un matériau élastique.

2. Butée arrière selon la revendication 1, dans laquelle la première surface de la première extension est au moins généralement arquée, la deuxième surface de la première extension étant au moins généralement plane.

3. Butée arrière selon les revendications 1 ou 2, dans laquelle le premier rebord s'étend généralement de manière perpendiculaire à la deuxième surface de la première extension, et/ou dans laquelle la première surface de la deuxième extension est au moins généralement arquée, la deuxième surface de la deuxième extension étant au moins généralement plane.

4. Butée arrière selon l'une quelconque des revendications 1 à 3, dans laquelle le deuxième rebord s'étend généralement de manière perpendiculaire à la deuxième surface de la deuxième extension, et/ou dans laquelle au moins une partie de la deuxième surface de chacune des première et deuxième extensions contacte au moins une partie d'un côté plan d'une bride (24) de la seringue.

5. Butée arrière selon l'une quelconque des revendications 1 à 4, dans laquelle les première et deuxième extensions s'étendent davantage de la deuxième surface que la jupe.

6. Combinaison, comprenant :
une seringue (14) incluant un cylindre (16) et une bride (24), la bride s'étendant vers l'extérieur du cylindre ; et
la butée arrière selon la revendication 1, configurée pour être fixée sur au moins une partie de la bride de la seringue, dans laquelle le premier rebord de la première extension s'étend vers l'extérieur de la deuxième surface de la première extension, au moins une partie du premier rebord contactant au moins une partie de la bride de la seringue, et dans laquelle le deuxième rebord de la deuxième extension s'étend vers l'extérieur de la deuxième surface de la deuxième extension, au moins une partie du deuxième rebord contactant au moins une partie de la bride de la seringue ; dans laquelle la combinaison comprend optionnellement en outre un piston plongeur (32) configuré pour être inséré dans au moins une partie du cylindre de la seringue, la butée arrière empêchant la séparation du piston plongeur de la seringue.

7. Combinaison selon la revendication 6, dans laquelle le piston plongeur inclut une extrémité distale (20) et au moins une saillie (40) s'étendant vers l'extérieur de celui-ci, l'extrémité distale étant configurée pour s'engager dans au moins une partie d'un bouchon, au moins une partie de la butée arrière contactant au moins une partie de la saillie du piston plongeur.

8. Combinaison selon les revendications 6 ou 7, dans laquelle la première surface de la première extension est au moins généralement arquée, la deuxième surface de la première extension étant au moins généralement plane.

9. Combinaison selon l'une quelconque des revendications 6 à 8, dans laquelle le premier rebord s'étend généralement de manière perpendiculaire à la deuxième surface de la première extension.

10. Combinaison selon l'une quelconque des revendications 6 à 9, dans laquelle la première surface de la deuxième extension est au moins généralement arquée, la deuxième surface de la deuxième extension étant au moins généralement plane.

11. Combinaison selon l'une quelconque des revendications 6 à 10, dans laquelle le deuxième rebord s'étend généralement de manière perpendiculaire à la deuxième surface de la deuxième extension.

12. Combinaison selon l'une quelconque des revendications 6 à 11, dans laquelle la bride de la seringue inclut un premier côté plan (24a), un deuxième côté plan diamétralement opposé (24b), un premier côté arqué (24c) et un deuxième côté arqué opposé (24d), et dans laquelle au moins une partie de la deuxième surface de chacune des première et deuxième extensions contacte optionnellement une partie de l'un des premier et deuxième côtés plans d'une bride de la seringue.
